# EUROPÄISCHE PATENTSCHRIFT

(11) **EP 2 117 567 B1**
(45) Veröffentlichungstag und Bekanntmachung des Hinweises auf die Patenterteilung: **18.06.2014**
(21) Anmeldenummer: 07721947.5
(22) Anmeldetag: 22.02.2007
(51) Int. Cl.: A61K 35/74, A61P 37/08

(54) **PROBIOTISCHE, GRAM-POSITIVE BAKTERIEN ZUR PROPHYLAXE, UNTERDRÜCKUNG ODER ELIMINIERUNG VON ALLERGISCHEN REAKTIONEN BEI MENSCHEN**
PROBIOTIC, GRAM-POSITIVE BACTERIA FOR THE PROPHYLAXIS, SUPPRESSION, OR ELIMINATION OF ALLERGIC REACTIONS IN HUMANS
BACTÉRIES GRAM POSITIF PROBIOTIQUES UTILISÉES POUR PRÉVENIR, ATTÉNUER OU SUPPRIMER DES RÉACTIONS ALLERGIQUES CHEZ L'HOMME

(43) Veröffentlichungstag der Anmeldung: 18.11.2009
(73) Patentinhaber: Schrezenmeir, Jürgen, Prof. Dr., 24114 Kiel (DE)
(72) Erfinder: Schrezenmeir, Jürgen, Prof. Dr., 24114 Kiel (DE)
(74) Vertreter: Pöhner, Wilfried Anton
(86) Internationale Anmeldenummer: PCT/DE2007/000333
(87) Internationale Veröffentlichungsnummer: WO 2008/101450

(56) Entgegenhaltungen:
- WO-A-01/97822
- DATABASE WPI Week 200680 Derwent Publications Ltd., London, GB; AN 2006-785256 XP002466730 & JP 2006 273852 A (MEIJI MILK PROD CO LTD) 12. Oktober 2006 (2006-10-12)
- DATABASE WPI Week 200030 Derwent Publications Ltd., London, GB; AN 2000-342512 XP002466731 & JP 2000 095697 A (ADVANCE CO LTD) 4. April 2000 (2000-04-04)
- POCHARD P ET AL: "Lactic acid bacteria inhibit TH2 cytokine production by mononuclear cells from allergic patients" JOURNAL OF ALLERGY AND CLINICAL IMMUNOLOGY, MOSBY - YEARLY BOOK, INC, US, Bd. 110, Nr. 4, Oktober 2002 (2002-10), Seiten 617-623, XP009022011 ISSN: 0091-6749
- PENG GUEI-CHENG ET AL: "The efficacy and safety of heat-killed Lactobacillus paracasei for treatment of perennial allergic rhinitis induced by house-dust mite." PEDIATRIC ALLERGY AND IMMUNOLOGY : OFFICIAL PUBLICATION OF THE EUROPEAN SOCIETY OF PEDIATRIC ALLERGY AND IMMUNOLOGY AUG 2005, Bd. 16, Nr. 5, August 2005 (2005-08), Seiten 433-438, XP002466729 ISSN: 0905-6157

## Beschreibung

Die Erfindung bezieht sich auf ein Arzneimittel- oder Nahrungs- oder Nahrungsergänzungsmittel sowie dessen Herstellung und Anwendung, das auch zur Prophylaxe, Unterdrückung oder Eliminierung von allergischen Reaktionen bei Menschen und/oder zur Verschiebung des TH1-TH2-Verhältnisses im menschlichen Körper in Richtung eines Anstiegs von TH1 und/oder eines Abfalls von TH2 dient, wie in den Ansprüchen 1-7 definiert.

Allergische Reaktionen auf Stoffe wie Blütenpollen (Heuschnupfen) oder andere pflanzliche Bestandteile, Katzenhaare und anderer Tierhaare, Staub mit darin enthaltenem Kot der Hausmilbe, Insektengift, Nahrungsmittel wie Milch oder Nüsse oder Duftstoffe und andere Bestandteile von Kosmetika sind weithin bekannt und ein zunehmendes Problem für immer mehr Menschen.

Die Ursache dafür ist, dass das körpereigene Abwehrsystem auf diese Stoffe so reagiert, als handele es sich um tatsächlich schädliche Eindringlinge wie z. B. Parasiten. Zudem ist das Ausmaß der Reaktion überhöht.

Im Ablauf dieser körpereigenen Abwehrreaktion spielen die weißen Blutkörperchen, die Lymphozyten, eine wesentliche Rolle. Eine Form von Lymphozyten sind die T-Lymphozyten oder Helferzellen (TH-Zellen), die zur Steuerung der Abwehrreaktionen verschiedene Botenstoffe, die Cytokine, absondern. Es gibt - **erstens** - Cytokine, die die allergische Reaktionen vermindern oder unterbinden und - **zweitens -** andere Cytokine, die entzündliche, also auch allergische Reaktionen auslösen, indem sie als Moderatoren auf Aktorzellen wir z. B. die Mastzellen, simulierend wirken. Nach dieser Wirkung werden alle TH-Zellen in zwei Gruppen aufgeteilt, nämlich die TH1- und die TH-2-Zellen. Die TH1-Zellen bilden antiallergische Cytokine, wie y -Interferon (IFN- γ) oder Interleukin-2 (IL-2). Die TH2-Zellen bilden Botenstoffe, die allergische Reaktionen auslösen, wie z.B. die Interleukine IL-3, IL-4, IL-5 und IL-10. Interleukin-4 stimuliert die Mastzellen zur Bildung des Antikörpers Immunglobulin Typ E (IgE) der bei Allergien in sehr großer Anzahl auftritt.

Das Verhältnis zwischen der Anzahl von TH1-Zellen und TH2-Zellen ist entscheidend für die Immun-Antwort des Körpers auf eingedrungene Erreger. Bei allergisch reagierenden Patienten ist es deutlich niedriger als bei gesunden Menschen. Bekannt ist, dass soeben geborene Babys oder Frühgeburten ebenfalls einen sehr niedrigen Wert haben, damit der mütterliche Organismus nicht fälschlicherweise Zellen des Kindes angreift.

Deshalb ist das TH1-TH2-Verhältnis eine wichtige Kenngröße eines jeden Menschen, die zunehmend auch einer breiteren Öffentlichkeit bekannt ist.

Es wird allgemein vermutet, dass Änderungen in der Darmflora und/oder das Fehlen von bakterieller Stimulation in frühester Kindheit durch allzu große Hygiene sowie durch ein Abnehmen der Infektionskrankheiten die Prädisposition für eine Abweichung des Wertes des Verhältnisses legen und damit für allergische Überempfindlichkeiten sorgen.

Schon im Kindesalter können deshalb beim Eindringen von Allergenen entzündliche Reaktionen, wie Schnupfen und geschwollene Schleimhäute bis hin zu erhöhter Köpertemperatur auftreten.

Es sind zahlreiche Arzneimittel bekannt, die die auftretenden Symptome bekämpfen oder unterdrücken. Sie haben den Nachteil, dass sie zum Teil sehr teuer sind, etliche unerwünschte Nebenwirkungen auslösen und kontinuierlich vom Patienten eingenommen werden müssen.

Auf aktuellem Stand der Technik nennen Pierre Pochard et al in ihrem Artikel "Lactic acid bacteria inhibit TH2 cytokine production by mononuclear cells fröm allergic patients" in der Publikation "Journal of Allergy and Clinical Immunology, Mosby Yearly Book, US Bd. 110, Nr. 4, Oktober 2002, S. 617 - 623, dass Lactobacillus als grampositive Bakterien die Produktion von den T_{H}2 cytokinen IL-4 und IL-5 unterdrücken und damit allergische Reaktionen reduzieren.

Auf diesem Hintergrund hat sich die Erfindung die Aufgabe gestellt, Wirkstoffe zu identifizieren, die der Prophylaxe, Unterdrückung oder Eliminierung von allergischen Reaktionen bei Menschen und/oder der Verschiebung des TH1-TH2-Verhältnisses im menschlichen Körper in Richtung eines Anstiegs von TH1 und/oder eines Abfalls von TH2 dienen.

Zur Lösung dieser Aufgabe schlägt die Erfindung ein Arzneimittel vor mit den im Anspruch 1 angegebenen Merkmalen.

Die Eigenschaft "probiotisch" erklärt der Brockhaus als das Zusammenleben von zwei Organismen zum Nutzen eines Partners ohne Schädigung des anderen. Im Falle dieser Erfindung nutzt dem menschlichen Organismus ein zweiter Organismus, nämlich das Bakterium. Dabei nimmt das Bakterium selbst keinen Schaden, bleibt also lebensfähig. Dazu gehört, dass bei oraler Verabreichung möglichst viele Bakterien den Magen mit seinen Säuren, Enzymen und anderen Verdauungswirkstoffen unbeschadet passieren.

Der Begriff probiotisch impliziert ebenfalls, dass nachteilige Wirkungen auf den Menschen vernachlässigbar oder zumindest sehr klein sind, sondern vorrangig Wirkungen eintreten, die als nützlich einzustufende.

Als **Probiotikum** wird eine Präparation aus Mikroorganismen verstanden, die bei Konsumierung ausreichender Mengen einen Gesundheitsfördernden Einfluss auf den Wirtsorganismus hat. Am längsten angewendet werden probiotische Milchsäurebakterien (Lactobacillus). Probiotika können als speziell zubereitete Lebensmittel oder in Form von Arzneimitteln dargereicht werden

Die gesundheitsfördernden Wirkungen von Probiotika sind jeweils stammspezifisch. Es sind probiotische Bakterienstämme bekannt, die auf der Basis von Evidenz basierter Medizin nachweislich die Lactose-Verdauung fördern, krankheitserregende Keime im Darm unterdrücken und Durchfall begrenzen oder unterdrücken.

Andere Bakterienstämme vermehren den Infektionsschutz, senken den Cholesterinspiegel und reduzieren das Risiko von Krebs im Dickdarm.

Diese Wirkungen sind vor der breiten Anwendung erfolgreich in vitro nachgewiesen worden. Von daher ist es legitim, die gesundheitsfördernde Wirkung dieser Erfindung mit einer im Labor durchgeführten Versuchsreihe zu begründen.

Mit den im folgenden geschilderten Versuchen wurde gemessen, welche Cytokine aus einer Fraktion von menschlichen Blutzellen, den PBMC - Blutzellen (Peripher Blood Mononuclear Cells), unter bestimmten Bedingungen freigesetzt werden. Diese Blutzellen wurden für eine Versuchsreihe aus dem Blut gesunder Menschen isoliert und für eine zweite Versuchsreihe aus dem Blut von allergischen Patienten, die gegen Hausstaub allergisch waren

Zur Stimulation wurde Staphylokokken-Enterotoxin Typ A (SEA) sowie in einer weiteren Reihe Dermatophagoides (Dpt) zugegeben, weil Hausstauballergiker meistens auch dagegen allergisch sind. Damit wird das Eindringen eines eine Allergie auslösenden Stoffes simuliert.

Stellvertretend für die T_{H}1 Reaktion wurde das γ- Interferon gemessen. Das T_{H}2-Muster wurde anhand der abgesonderten Interleukine 4 und 5 (IL-4 und IL-5) erfasst.

Unter den Faktoren, die möglicherweise an der gewachsenen Verbreitung von allergischen Krankheiten beteiligt sind, wird vermutet, dass die Modifikation der Darmflora oder das Fehlen von bakterieller Stimulation in der Kindheit beteiligt ist. T_{H}2-Zytokine steigern die Produktion von IgE und stimulieren Mastzellen. Hingegen tragen γ-Interferone (IFN-γ), ein TH1-Zytokin, dazu bei die IgE Synthese zu unterdrücken. Auf diese Weise kann das Ungleichgewicht in dem Ausdruck "TH1 zu TH2" zur Auslösung und Aufrechterhaltung von allergischen Krankheiten beitragen. Lactobacillus Bakterien, die zum natürlichen Mikroflora des Darminneren gehören, sollen die Häufigkeit und die Schwere von allergischen Manifestationen vermindern und die TH1/TH2 Antwort modulieren. Der Funktionsmechanismus bleibt noch aufzuklären.

Ziel: Wir haben das Versuchsziel gehabt, den Einfluss von probiotischen Bakterien auf die Herstellung von Typ TH1 und TH2-Zytokinen von gesunden Menschen und Patienten mit Allergien gegen Hausstaubmilben zu bestimmen und die molekulare Grundlage der Einflüsse von bakterieller, genomischer DNA auf die TH1/TH2 Antwort auf Staphylokocken Enterotoxin A (SEA) und Dermatophagoides Pteronyssinus (DPT) aufzuklären und mit den Einflüssen von lebenden Bakterien zu vergleichen.

### Methoden:

PBMC's von Patienten mit Allergien gegen Hausstaubmilben im Vergleich zu solchen von gesunden Spendern wurden für 24 und 48 Stunden inkubatiert mit oder ohne SEA und DPT Allergenzien. Der Einfluss einer Vorinkubation mit lebenden, probiotischen Bakterien wie auch der Einfluss ihrer genomischen DNA, die simultan den Kulturen hinzugefügt wurde und für 24 Stunden ausgebrütet wurde, wurde durch die Messung der TH1/TH2 Cytokinproduktion bewertet.

### Ergebnisse:

Die geprüften, lebensfähigen, gram-positiven, probiotischen Bakterien und ihre genomische DNA zeigten, dass sie die SEA- und DPT-stimulierte Absonderung von TH2 Cytokinen (EL4 und EL5) unterdrückten und die Stimulation von IFN- γ erweiterten. Dieser Effekt war sowohl von der Dosierung als auch vom gewählten Bakterienstamm abhängig. Keine signifikante Hemmung wurde von der Kontrolle, gram-negativen Escherichia coli TG1 ausgelöst. Probiotische Bakterien reduzierten die Produktion von IL-4 Cytokinen von allergischen PBMCs insbesondere nach einer erneuten Stimulierung mit SEA und DPT sogar noch wirkungsvoller als von gesunden Menschen. Hingegen war eine IL-5 Hemmung bei gesunden Subjekten deutlicher ausgeprägt. Bakterielle DNA unterdrückte die Freisetzung von IL-4 und IL-5 ebenfalls, jedoch nur in einem etwas geringerem Umfang. Die Hemmung von EL4 war bei PBMCs von allergischen Subjekten mehr ausgeprägt als bei gesunden Subjekten wo hingegen dieses bei EL5 gegeben war.

### Zusammenfassung:

Die TH1/TH2 Antwort auf Allergenzien, moduliert durch die geprüften probiotischen Bakterien, ebenso wie ihre genomische DNA, zeigte eine Anti-TH2 Aktivität. Folgerichtig können verschiedene Stämme von probiotischen Bakterien und ihre genomische DNA nützlich bei der Vorbeugung von allergischen Krankheiten sein.

### 1.Einführung:

Die Lehre von den Krankheitsursachen allergischer Krankheiten bleibt unklar, sogar wenn menschliche, experimentelle Studien anzeigen, dass genetische Vorbedingungen, die Immunantwort der Darmschleimhaut und enteritische Bakterien zu der Pathogenese von allergischen Krankheiten beitragen. Einige Studien schlagen vor, dass TH2 Cytokine, insbesondere IL-4, IL-5, IL- 9 und IL-13, lebenswichtige Rollen in der Pathogenese spielen, indem sie die Produktion von IgE regeln und Mastzellen stimulieren. Die Produktion von IL-4, IL-5, IL-9 oder IL-13 durch TH2 Lymphoziten auf einem hohen Niveau kann eine entscheidende Rolle in der Entwicklung und dem Verlauf von allergischen Antworten spielen. Im Gegensatz dazu hat IFN-γ, ein sogenanntes TH1 Cytokin, die Fähigkeit, die IGE-Synthese zu unterdrücken. Defekte IFN-γ Ausdrücke sind oft in Verbindung gebracht worden mit IgE vermittelten Allergien. Die Disregulation in dem Verhältnis von TH1/TH2 Cytokinen kann zu einem großen Teil für die Auslösung und Aufrechterhaltung von allergischen entzündlichen Prozessen bei Krankheiten wie z. B. bronchialem Asthma oder atobischer Dermatitis verantwortlich gemacht werden.

Es ist vorgeschlagen worden, dass besondere TH2 Cytokinprofil von neugeborenen Babys, das Altern, das normalerweise das TH1/TH2 Verhältnis herabsetzt, moderne Hygieneregeln, intensive Sterilisation von Nahrungsmitteln und/oder die Veränderungen der Darmflora von neugeborenen Babys, die durch das Füttern nach künstlichem Rezept verursacht ist, eine herausragende Rolle bei den Änderungen des TH1/TH2 Gleichgewichtes spielen. Das gewachsene Wissen auf diesem Gebiet hat die Grundlage für eine Anzahl von neuen Therapien bereitgestellt. Ein Ansatz ist es, einfach probiotische Bakterien zu nutzen, um diejenigen Cytokine zu vermehren, die nicht ausreichend gebildet werden oder diejenigen Cytokine zu vermindern, die in zu großem Umfang produziert werden, weil probiotische Bakterien die TH1/TH2 Balance modulieren.

Probiotische Bakterien sind allgemein als sicher eingestuft und von Menschen oder Tieren verzehrt worden. Die Bakterien Lactobacillus und Bifidobacterium sind die am weitesten verbreiteten Mikroorganismen im menschlichen Magen-Darm-Trakt. Das Interesse an der immunstimulierenden Wirkung von probiotischen Bakterien wächst, insbesondere an der antiallergischen Wirkung. Die wichtige Rolle von Bakterien bei allergischen Krankheiten legt die Möglichkeit nahe, diese Abweichungen zu verhüten oder zu behandeln, indem die Mikroflora des Darmes durch probiotischen Behandlung verändert wird. Die probiotischen Bakterien können direkt oder indirekt durch die Modulation der endogenen Flora oder des Immunsystems wirken. Es wurde vorgeschlagen, dass der neue Ausdruck "Immunobiotics" benutzt wird, um Bakterien zu benennen, die Gesundheit über das mukosale Immunsystem einbringen, im Gegensatz zu solchen mit nur lokal begrenzter Wirkung. Verschiedene Immunantworten auf probiotix sind berichtet worden, wie die Absonderung von entzündungsfördernden Cytokinen, die Vermehrung von Lymphorcyten und die Bildung von Stickoxiden. Weiterhin hat sich gezeigt, dass ganze Zellen, einige lösliche Komponenten, die von LGG oder der DNA von LGG abgesondert wurden, Komponenten der Zellwand wie z. B. Peptidoglykan oder Lipoteichionische Säuren von Laktobacillen entzündliche Immunantworten auslösen und immunobiotische Aktivitäten zeigen. Es wurde demonstriert, dass nicht nur lebende Bakterien, die in den Darmtrakt verabreicht werden, sondern auch isolierte, probiotische DNA aktiv ist, sogar wenn sie subcutan injiziert wird.

Es wurde berichtet, dass unvergällte CpG Leitelemente in bakterieller DNA mitogen für B-Zellen von Mäusen sind und die Herstellung von entzündungsbildenden Cytokinen durch Makrophagen und dentritische Zellen (DC) auslösen. Und CpG Oligodeoxinukleotide (ODNs) und bakterielle DNA die Produktion von Interleukin 6 (EL-6), Interleukin 12 (EL-12) und Interpheron-γ (IFN-γ) durch B-Lymphozyten und natürliche Killerzellen von Mäusen auslösen. Weiterhin induziert die CpG DNA die Vermehrung von B-Zellen und die Teilungen von unbeeinflussten und aktivierten T-Zellen in T-Helferzellen 1 und 2. Bestimmte CpG ODNs (D-Typ) sind besonders leistungsfähig, um NKZellen zu aktivieren und die Produktion von α-Interferon (IFN-α) durch plasmacytoide, dentritische Zellen auszulösen, wohingegen andere ODNs (K-Typ) besonders leistungsfähige Aktivatoren von BZellen sind. Es hat sich vor kurzem gezeigt, dass der abgabenähnliche Rezeptor 9 (TLR9) eine kritische Rolle bei der Auslösung einer zellularen Aktivation durch CpG DNA spielt.

Experimente die in Mäusen ausgeführt sind, welche auf Ovalbumin sensibilisiert sind, zeigten, dass nach Verabreichung von Milchsäurebakterien in den Magen die besonderen IgE- und TH2-Profilabhängigen, entzündlichen Antworten unterdrückt wurden. Weiterhin schlagen neueste Berichte vor, dass der Lactobacillus Rhamnosus GG die Symptome vom allergischen Krankheiten in menschlichen Subjekten reduzieren kann, ebenso wie er angeborene Immunantworten auslösen kann durch die Aktivierung von Transcriptionsfaktoren, die in die Signalisierungsfunktion von Cytokinen eingebunden sind. Die Gabe dieses Bakterienstammes an stillende Mütter und neugeborenen Babys führte zur Unterdrückung des Risikos von atopischen Exzemen bei Babys. In-Vitro-Experimente haben gezeigt, dass die Stimulierung von PBMCs oder Monocyten von gesunden Spendern durch eine Vielfalt von Milchsäurebakterien die Absonderung von IL-12 induziert, das ein lebenswichtiges Pro-TH1-Cytokin ist, welches an der Kontrolle der Entwicklung von allergischen Krankheiten beteiligt ist. Die Funktionsmechanismen, durch die LAB die Produktion von TH2 Cytokinen beeinflusst, die für die Initiation der Entwicklung von allergischen Krankheiten verantwortlich sind, bleibt noch zu benennen. Unter Beachtung des Vorhandenseins von unvergällten DNA-Sequenzen (CpG Leitelemente) in den Chromosomen der DNA von Bifidobacterium und Lactobacillus in dieser Studie entschieden wir uns, die Wirkung von vier Stämmen der Bakterien Lactobacillus und Bifidobacterium ebenso wie ihre chromosomalen DNA auf IL-4, IL-5 und IFN-γ Produktion durch SEA und DPT stimulierte PBMCs von gesunden und allergischen Probanden zu prüfen. Die untersuchten Bakterien Lactobacillus und Bifidobacterium zeigten Anti-TH2-Aktivitäten und das Pro-TH1-Cytokin, IFN-γ.

### 2. Methoden und Material

### 2.1. Bakterienkultur

Die Bakterienstämme, die in dieser Studie gebraucht wurden, sind die folgenden:
Lactobacillus- rhamnosus GG (92164), Lactobacillus gasseri (PA 16/8), Bifidobacterium bifidum (MG 20/5), Bifidobacterium longum (SP 07/3) und LgsB.bB.I (eine Mischung von Lactobacillus gasseri, Bifidobacterium bifidom und Bifidobacteriumg longum). Bifidobacterium und Lactobacillusstämme wurden herangezogen (eine 0,02 % inoculum von Stämmen, die bei minus 80°C in 30 % Glycerol gelagert wurden) in anaerober Weise (Anaerobisches System, MACS - VA500 workstation mit Luftschleuse und Luftschleuse, Don Whitley Scientific Limited UK in einem MRS Medium (MRS; Merck, Darmstadt, Deutschland), ergänzt mit 0,05 % L-cystein bei 37°C für 16 Stunden. Vor der Ernte und der Waschung in PBS wurden fortlaufende Lösungen von frisch präparierten Kulturen auf Platten aufgebracht, die MRS-agar Medien enthielten und zur Zählung kultiviert. Die Zählung der Kolonie bildenden Einheiten (CFU ml⁻¹) der probiotischen Bakterien Gruppen wurde durch das Aufbringen von wiederholten 10-fachen Lösungen auf die Platten abgeleitet. Die Platten wurden anaerob bei 37°C für 24-48 Stunden ausgebrütet. Die Bakterien wurden dann 3 mal mit PBS gewaschen und auf die entgültige Konzentration von 10¹⁰, 10⁷ und 10¹ CFU ml⁻¹ eingestellt. Die bakterielle Suspension wurde bei - 80°C in einer MRS Lösung gelagert, die 30 % Glycerol enthielt. Für alle Bakterienstämme wurden normale Wachstumskurven hergestellt durch die Aufzeichnung von OD600 vs agar Platten-Zählungen von frisch präparierten, wiederholt gelösten Kulturen. Um die Zählungen von lebensfähigen Bakterien in frisch präparierten Kulturen zu berechnen, wurden die Kurven mit logarithmischen Ausdrücken ausgestattet, die alle Werte über 98,5 % gewannen (Daten nicht gezeigt). Gram-negative E. coli TG1 (Produktnummer BU-00035) wurde von Maxim Biothec Inc. gekauft und wuchs im LB-medium für 18 Stunden bei 37 °C und wurde wie oben geerntet.

### 2.2. Die Vorbereitung von genomischer DNA aus Bakterien

Genomische DNA von reinen Kulturen probiotischer Bakterien wurde durch die Extraktion mit Phenol/Chloroform/Isomyl Alkohol (25:24:1) gereinigt. Um einen vollständigen Zellaufschluss zu erhalten, wurde die Methode geringfügig modifiziert, indem die enzymatische Lyse von 2 auf 7 Stunden verlängert wurde. Anschließend wurde die DNA ausgefällt, mit kaltem (-20°C) 95 % Ethanol sterilisiert und in doppelt destilliertem Wasser (ddH2O) gelöst. Die Konzentration und die Reinheit von allen DNA Präparationen wurde durch die Messung der OD ₂₆₀ Absorbierung, bzw. des OD _{260/280} und 260/230 Verhältnisses abgeleitet. Es wurden nur DNAs mit einem OD _{260/280} Verhältnis >1,8 und 260/230 ≥ 2 benutzt. DNAs wurden von Endotoxin mit TritonX-114D gereinigt und auch auf ihren Gehalt an Lipopolysaccharide (LPS) untersucht durch die Nutzung der Limulus-Amebocyten-Untersuchung (QCL-1000, CAMBREX, Deutschland). Der LPS Gehalt betrug weniger als 0,01 U Endotoxin µg^{-1..}
Um eine Bioaktivität von LPS oder anderen bakteriellen Kontaminanten in den DNA Präparationen zu bestimmen, wurde die DNA mit DNase I (Sigma) abgebaut. Es gab keine nachweisbare Unterdrückung von Cytokinen durch PBMCs unterhalb des Basisniveaus, wenn die abgebaute DNA mit einer Konzentration von 75 µg ml⁻¹ hinzugefügt wurde (gemessen vor der DANN-Aufschlussbehandlung).

### 2.3. PBMC Isolation und Kultur

### 2.3.1. Vorinkubation von PBMCs mit probiotischen Bakterien und weitere Stimulation mit SEA und Dpt

Acht allergische Patienten und acht gesunde Spender wurden für die Studie rekrutiert. Von allen Probanden wurde eine mündliche und schriftliche Zustimmung vor ihrer Registrierung zu dieser Studie in Übereinstimmung mit der Ethik-Komission der Universität Kiel über den Einsatz von menschlichen Probanden in der Forschung eingeholt. Venöses Blut wurde von den Spendern in heparinisierte Vakutianer geleitet und 1:1 mit 0,9 % NaCl verdünnt. Dann wurden frische Periphere Blut Mononukleare Zellen (PBMCs) aus heparinisiertem, verdünntem Blut durch Zentrifugieren nach steigender Dichte isoliert (1,077 g ml⁻¹) (Lymphoprep, AXIS-SHIELD PoC AS, Oslo, Norwegen). Die Zellen wurden im RPMI-1640 Kulturmedium (Sigma, München, Deutschland), ergänzt mit 10 % (v/v) hitzeaktivierten (56°C, 1 Stunde) fetalem Rinderserum, Gentamicin (50 µg ml⁻¹) (Sigma), penicillinstreptomycin (1%) und Sodium Pyruvatlösung (0,23 mmol l⁻¹) (Sigma) (bestellt als vollständiges Medium) in eine Emulsion eingebracht. Alle Komponenten wurden Endotoxin geprüft eingekauft, wie es von LAL bestimmt ist. Die Zellen wurden in dem vollständigem Medium in einer Konzentration von 2×10⁶ Zellen ml⁻¹ in Gefäßen mit 24 Kammern kultiviert. Gleichzeitig wurden zwecks Stimulation vier Stämme von lebenden, probiotischen Bakterien und einem Gram-negativen Bakterium (E-coli TG1) zu den Kulturen hinzugefügt. Wie für bakterielle Zählungen erforderlich, waren die probiotischen und die anderen Bakterien zum Zeitpunkt der Hinzugabe zu den Kulturen lebensfähig. Die Zellen, die allein mit dem Medium kultiviert waren, dienten als nicht stimulierte Kontrolle. Die Versuche zur Abhängigkeit von der Dosierung, ausgeführt für IL-4 und IL-5 als Kulturen mit einem Endvolumen von 200 µl/Kammer wurden in flachbödigen 96-Kammern-Mikrotiter-Platten (Nune, Roskilde, Dänemark) vorbereitet mit 2x10⁶ mononuclearen Zellen und 5x10⁴, 5x10⁵, 2x 10⁶, 5x10⁶, 2x10⁷ und 5x10⁷ Bakterien/ml entsprechend 0,025, 0,25, 1, 2,5, 10 und 25 Bakterien pro mononucleare Zelle. Sie zeigten an, dass die maximale Unterdrückung mit 2x10⁷ CFU von Bakterien/ml entsprechend einem Verhältnis von 10:1 (Bakterien zu PBMC) beobachtet wurde. Diese Konzentration wurde bei weiteren Experimenten benutzt. Das endgültige Verhältnis zwischen PBMC und Bakterien (Bakterien zu PMBC Verhältnis) war 10:1 für gesunde Spender und für allergische Patienten. Zur Kontrollbehandlung wurde nur Kulturmedium zu der PBMC Lösung hinzugegeben. Danach und nach drei Stunden Bebrütung bei 37°C wurden die PBMCs weiter stimuliert mit SEA (2µg ml ⁻¹) oder Dpt (2000SQ-E ml⁻¹ äquivalent zu 2 µg Gabe ml⁻¹) und in einem 5 % CO₂ befeuchteten Inkubator bei 37°C für 48 Stunden bebrütet. Alle Experimente wurden doppelt ausgeführt.
Nach einer Bebrütung von 48 Stunden wurde das Kulturmedium bei 4 °C für 20 Minuten bei 1000xg zentrifugiert. Der zellfreie Überstand wurde mittels passieren durch einen Filter mit einer Porengröße von 0,2 µm sterilisiert (Millipore, Deutschland) und bei -80°C bis zum Gebrauch gelagert. Die Lebensfähigkeit der Zellen wurde vor und nach der Bebrütung mit Bakterien durch die Ausschließung mit Trypan Blau bestimmt.

### 2.3.2. Stimulation mit SEA, LPS und genomischer DNA

Frische PBMC wurden aus heparinisiertem, peripherem Blut von vier gesunden Spendern durch Zentrifugierung nach steigender Dichte (1,077g ml⁻¹) (Lymphoprep, AXIS-SHIELD PoC AS, Oslo, Norwegen) isoliert. Die Zellen wurden in einem RPMI 1640 Kulturmedium (Sigma, München, Deutschland) mit 10 % (v/v) hitzeaktivierten (56°C, 1 Stunde) fetalem Rinderserum, Gentamicin (50 µg ml⁻¹) (Sigma), Penicillin-Streptomycin (1 %) und Sodiumpyruvatlösung (0,23 mmol l⁻¹) (Sigma) (vollständiges Medium). Alle Komponenten wurden Endotoxin getestet gekauft. Alle Versuche zur Abhängigkeit von der Dosierung, ausgeführt für IL-4 und IL-5 als Kulturen mit einem letztendlichen Volumen von 200 µl/Kammer wurden in einer flachbödigem 96-Kammer Mikrotiterplatte (Nune, Roskilde, Dänemark) mit 2x10⁶ mononuklearen Zellen und 5, 10, 25, 50, 75 und 100 µg genomischer DNA pro ml aufgesetzt. Sie zeigten an, dass die maximale Unterdrückung mit 75µg DNA ml⁻¹ zu beobachten war. Diese Konzentration wurde im weiteren Experiment benutzt. Die Zellen wurden in einem vollständigen Medium in einer Konzentration von 2x10⁶ Zellen ml⁻¹ in einer 24-Kammer-Platte (Nunc, Roskilde, Dänemark) kultiviert. Gleichzeitig wurde genomische DNA (75µg ml⁻¹) von probiotischen Bakterien, genomische DNA vom Kalb (75µg ml ⁻¹, Sigma, München, Deutschland), SEA (2µg ml⁻¹), LPS von E. coli (20ng ml⁻¹, Sigma, München, Deutschland) der Kultur hinzugefügt und bei 37°C, 5 % CO₂-Befeuchtung für 24 Stunden bebrütet. Alle Experimente wurden 3-fach ausgeführt. Nach 24 Stunden Inkubation wurden die zellfreien Überstände gesammelt und bei 1000 xg für 20 Minuten, 4°C zentrifugiert und sterilisiert mittels Passieren durch einen Filter mit 0,2 µm Poren (Millipore, Deutschland) und bei -80°C in Aliquoten bis zur Analyse gelagert. Die Lebensfähigkeit der Zellen wurde vor und nach der Inkubation mit genomischer DNA durch die Ausschließung mit Trypan Blau ermittelt. Bei allen Experimenten waren 95 - 98 % der PBMCs lebensfähig. Die Experimente zur Abhängigkeit von der Dosierung, die für jedes Cytokine ausgeführt wurden, zeigten an, dass die maximale Unterdrückung bei einer Konzentration von 75 µgml⁻¹ beobachtet wird. Diese Konzentration wurde bei den weiteren Experimenten genutzt.

### 2.4. Untersuchung der Cytokine mittels Enzyme basierter immunosorbenter Untersuchung (ELISA)

Der Gehalt an IL-4, IL-5 und IFN-γ wurde in den zellfreien Überständen mithilfe von einer besonderen ELISA (BD OptEIA^{™} set menschliches IL-4, 5 und IFN-γ Heidelberg, Deutschland) quantifiziert. Die Nachweisgrenze der Untersuchung war 0,5 pg ml⁻¹ für IL-4, 0,5 pg ml⁻¹ für IL-5 und 1 pg ml⁻¹ für IFN-γ. Die optischen Dichtheitswerte der Proben wurden bei 450 und 570 nm auf einem ELISA Plattenleser abgelesen. Die Experimente wurden wenigstens doppelt wiederholt und dreifach ausgeführt.

### 2.5. Statistische Analysen

Die Daten des Versuchs wurden mittels ± S.E.M ausgegeben und eine nicht parametrisierte, statistische Analyse mit dem t-Test ausgeführt. P-Werte von weniger als 0,05 wurden als statistisch signifikant betrachtet.

### Ergebnisse

Lactobacillus und Bifidobacterium unterdrücken IL-4 und IL-5 und lösen eine IFN-γ Produktion durch SEA oder Dpt-stimulierte PBMCs von gesunden Spendern aus. Es wurde gezeigt, dass Streptokokken Superantigene einen hohen Gehalt von IL-4 und IL-5 aus PBMCs von gesunden Spendern auslösen und das durch Hitze abgetötete Milchsäurebakterien in der Lage waren, die Sekretion eines Typ 2 Cytokin-Profiles zu unterdrücken. Weiterhin hat eine Studie gezeigt, dass allergische Patienten einen erhöhte Gehalt an IL-4 und IL-5 hatten. Dermatophagoides Pteronyssinus, Dpt (mit 83,8%) und Dermatophagoides farinae, Df (mit 78,4%) sind die am meisten verbreiteten, kausativen Allergene bei Patienten mit allergischer Rhinitis. Jetzt haben wir diese Beobachtungen t mit einem anderen Superantigen bestätig: Staphylokokken Enterotoxin A (SEA) und Dpt.

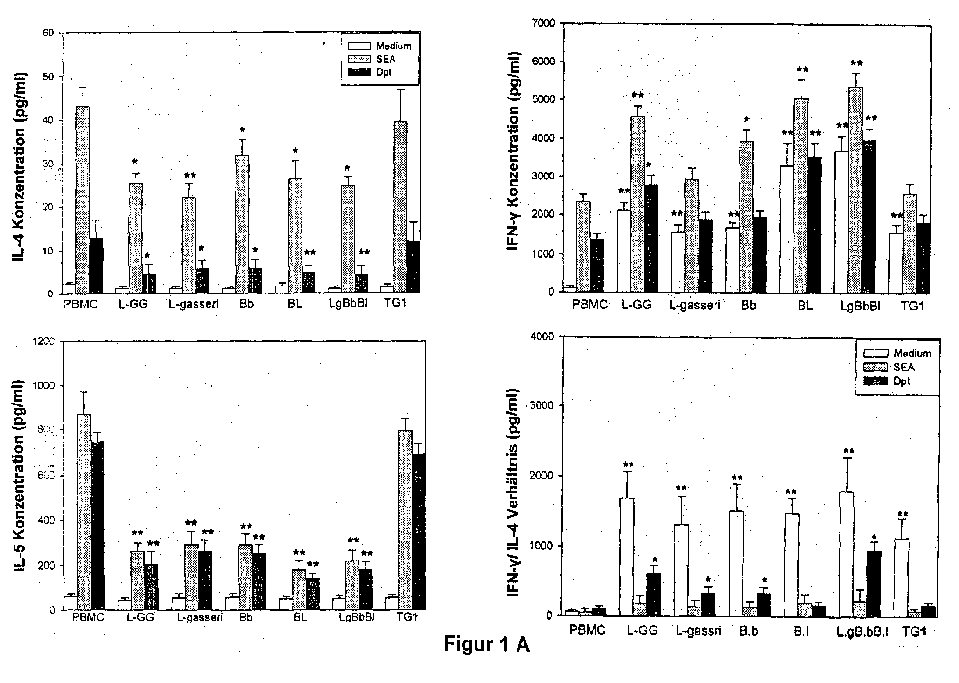

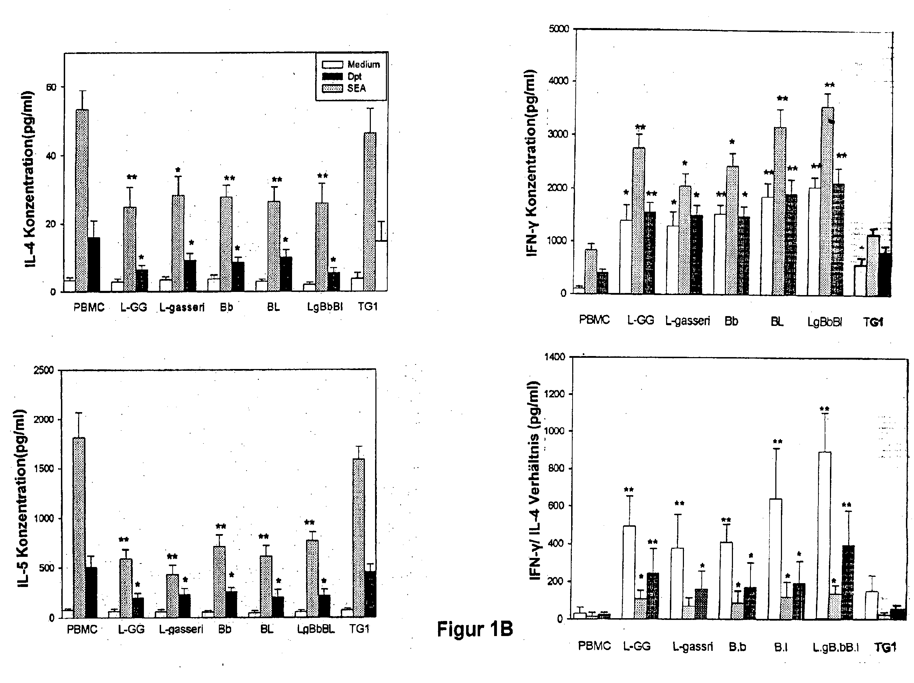

Wenn lebende Stämme von den Bakterien Lactobacillus und Bifidobacterium mit PBMCs vorinkubatiert wurden, wurde die sich daraus ergebende Produktion von IL-4 durch SEA und Dpt in hohem Maße vermindert, verglchen mit dem, was durch die positive Kontrolle ausgelöst worden ist (keine Vorinkubation mit den Lactobacillus und Bifidobacterium). Interessanter Weise wurde keine signifikante Unterdrückung beobachtet wenn PBMCs mit den grammnegativen Kontrollstämmen TG1 vorinkubatiert wurde (Figur 1).

Obwohl weder die vier Stämme Lactobacillus und Bifidobacterium noch TG1 eine Basis-IL-4 Produktion auslösten, induzierten alle 4 Stämme die Produktion von IFN-γ. Wenn PBMCs mit SEA und Dpt stimuliert wurden, wurde zusätzlich der Gehalt an IFN-γ Freisetzungen stark erhöht. Ein synergistischer Effekt wurde nicht nur mit den Bakterien Lactobacillus und Bifidobacterium beobachtet, sondern auch mit TG1. Folglich erscheinen Lactobacillus und Bifidobacterium in der Lage zu sein, sowohl die Absonderung von TH1 als auch von TH2 Cytokinen in entgegengesetzter Weise zu beeinflussen.

Die Produktion von Cytokinen hängt ab von der Zeit und der Dosierung.

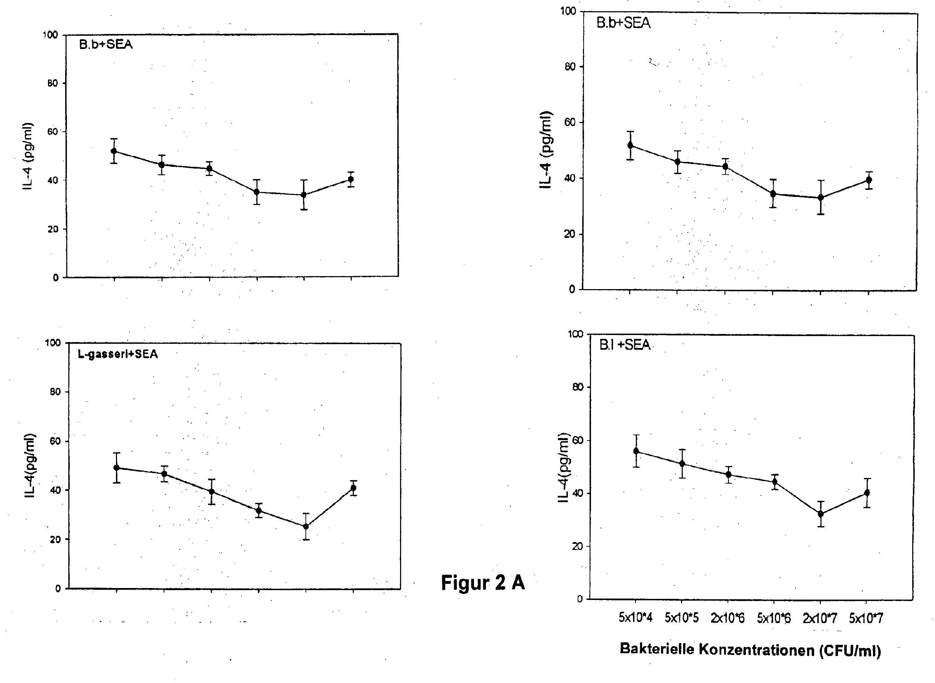

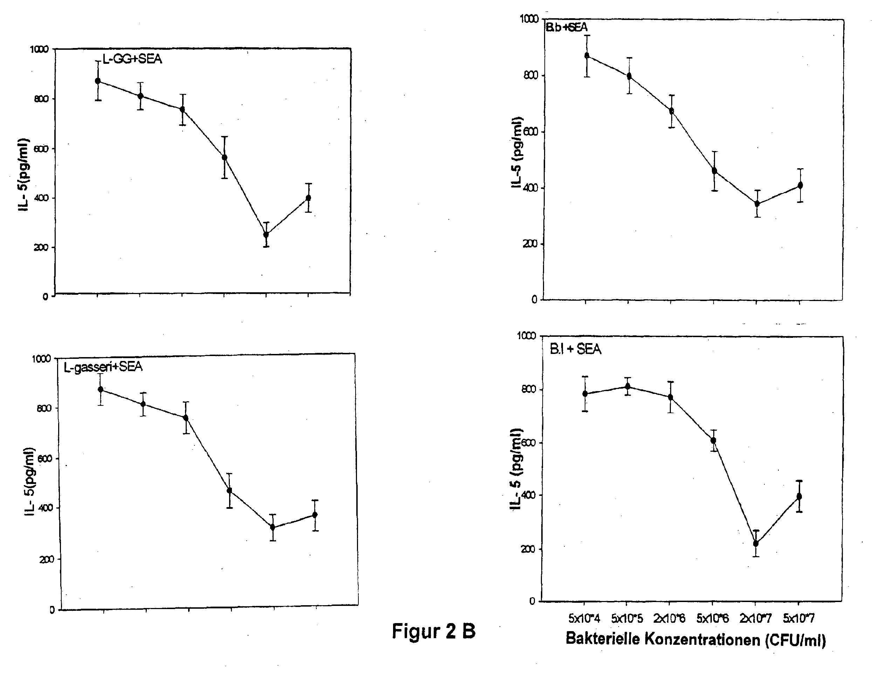

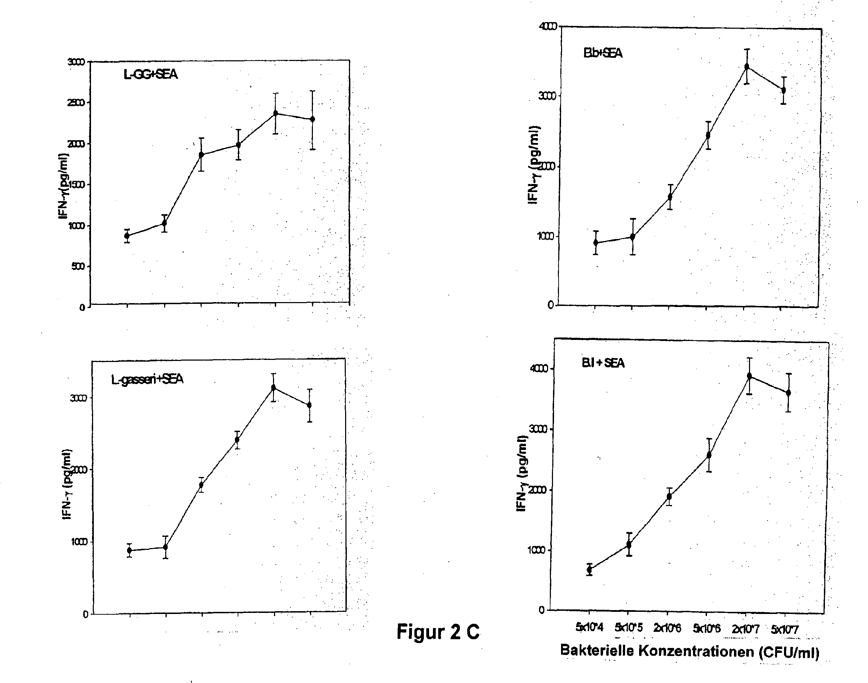

Weitere Experimente, die mit vier Lactobacillus- und Bifidobacteriumstämmen ausgeführt wurden, zeigten, dass die Unterdrückung von TH2 Cytokinen von der Zeit und der Dosis abhängen. Wenn PBMCs vor der Hinzugabe von SEA mit probiotischen Bakterien stimuliert wurden, nahm der wachstumshemmende Einfluss auf die TH2 Cytokinproduktion mit dem Verhältnis von Bakterien zu PBMC zu. Die maximale Wachstumshemmung wurde bei 2x10⁷ CFU von Bakterien ml⁻¹ entsprechend einem Verhältnis von 10:1 (Bakterien zu BMC) (Figur 2, A und B) beobachtet.

Dieser Effekt wurde ebenso für die Produktion von IL-4 und IL-5 beobachtet. Zum Beispiel wurde für die L-gasseri Stämme der Prozentsatz der Wachstumshemmung von der IL-4 Produktion in Antwort auf SEA von 40,19 % ± 3% (1:1 Verhältnis) auf 54,22 % ± (10:1 Verhältnis) angehoben. Der Prozentsatz der Unterdrückung der IL-5 Produktion wuchs von 43,77 % ± 8 % (1:1 Verhältnis) auf 73,17 % ± 5 % (10:1 Verhältnis). Zusätzlich erhöhte sich der Prozentsatz der Hemmung der IL-4 Produktion in Antwort auf Dpt. bei PBMC von gesunden Spendern von 34,61 % ± 4 % (1:1 Verhältnis) auf 47,33 % ± 5 % (10:1 Verhältnis). Der Prozentsatz der Wachstumshemmung der IL-5 Produktion wuchs von 45,72 % ± 4 % auf 77,59 % ± 6 %. Im Gegensatz zu TH2-Cytokin induzierten unsere getesteten Stämme eine Grundproduktion von IFN- γ, die von dem Bakterien-/Zellenverhältnis abzuhängen scheint. Es ist bemerkenswert, dass gezeigt werden konnte, dass probiotische Bakterien in hohem Maße die Produktion von IFN-γ als Antwort auf eine Stimulation mit SEA beeinflussen (Figur 1 und 2, C)

### Zeitabhängige, wachstumshemmende Wirkung von lebenden probiotischen Bakterien auf die Produktion von IL-4 und IL-5.

PBMCs von gesunden Spendern (n = 4, bei 2x1 0⁶ml⁻¹) wurden mit SEA (2 µg/ml) stimuliert. Mononuclearzellen wurden entweder für 1,3 und 5 Stunden mit L-GG (10:1 Verhältnis) vor Inkubation vor der SEA-Stimulierung vorinkubatiert oder sie wurden simultan stimuliert mit den L-GG Stämmen und das Superantigen oder L-GG wurde 1,3 und 5 Stunden nach der SEA Stimulierung hinzugegeben und die Zellkulturen wurden über eine Periode von 24,48 und 72 Stunden vorbebrütet. Zu den genannten Zeitpunkten wurden die zellfreien Überstände geerntet und nach Zentrifugierung und Sterilisation bei - 20°C gelagert. Die Konzentration an IL-4 und IL-5 wurde gemessen und zeigte, dass eine maximale Wachstumsunterdrückung beobachtet wurde bei einer 3-stündigen Vorinkubation von PBMCs mit lebenden Bakterien vor der Stimulation mit SEA. Eben so wurde eine maximale Wachstumshemmung bei einer 48-stündigen Inkubation beobachtet (Daten nicht gezeigt). Diese Zeitspanne wurde in den mweiteren Experimente gebraucht.

### Lactobacillus und Bifidobacterium unterdrücken die Produktion von TH2 Cytokinen von allergenstimulierten PBMCs von allergischen Patienten

Die Fähigkeit von Lactobacillus und Bifidobacterium, das Ungleichgewicht zwischen TH1- und TH2-Cytokinen zu korrigieren, wurde als nächstes in einem noch relevanterem Modell der Stimulation durch Allergene bewertet. Wenn PBMCs von Patienten (allergisch auf D. pteronyssinus) mit verschiedenen, lebensfähigen Stämmen von Lactobacillus und Bifidobacterium vorbebrütet wurden, wurde die Produktion von IL-4 und IL-5, ausgelöst nach einer spezifischen Stimulation mit D. pternonyssinus Allergenen, in großem Umfang reduziert und zwar in einer Weise, die von dem Bakterien-/Zellverhältnis abhängt. Diese Wirkung auf IL-5 war unabhängig von der Stammart der untersuchten probiotischen Bakterien. Die Unterdrückung von IL-5 entsprach beispielsweise bei B.b. 57,31 % ± 4,52 % und bei B.I. 63,77 % ± 5 % (Figur 1). Weiterhin wurden ähnliche Effekte beobachtet, wenn PBMCs von allergischen Patienten mit SEA stimuliert wurden. In diesem Fall, obwohl die IL-4 und IL-5 Produktion sehr hoch war im Vergleich mit der Produktion die mit D pteronyssinus beobachtet wurde (Figur 1), reduzierten die getesteten probiotischen Bakterien die Absonderung von IL-5 um 71,29 % ± 4,10 % für L-GG und 77,63 % ± 3,52 % für L-gasseri (Figur 1). Koinkubation mit Lactobacillus und Bifidobacterium erhöhte die Produktion von IFN-γ in hohem Maße (Figur 1). Auf diese Weise durch die Reduzierung der Produktion von TH2 Cytokinen und durch die Verstärkung der IFN-γ Produktion von PBMCs von allergischen Patienten scheinen Lactobacillus und Bifidobacterium eine Anti-TH2-Aktivität aufzuweisen. In diesem Zusammenhang ist es interessant zu bemerken, dass die wachstumshemmende Wirkung von Lactobacillus und Bifidobacterium auf die IL-4 Produktion durch PBMCs von allergischen Probanden höher ist als bei gesunden Probanden. Im Gegensatz dazu ist die wachstumshemmende Wirkung dieser Bakterien auf die IL-5 Produktion durch PBMC von gesunden Spendern höher als bei allergischen Probanden (Figur 1). Im Vergleich dazu war der wachstumshemmende Effekt von B. I. und LgsBbBI auf IL-4 Produktion durch Dpt stimulierte PBMC von allergischen Probanden höher als von gesunden Probanden (p<0,05) und die hemmende Wirkung von L-GG, B. b und B. I auf IL-4 Produktion durch SEA-stimulierte PBMC von allergischen Probanden war höher als bei gesunden Probanden (p<0,01). Im Gegensatz dazu war der wachstumshemmende Effekt von L-Gassery, B.b und B.I auf die IL-5 Produktion durch Dpt-stimulierte PBMC von gesunden Probanden größer als bei allergischen Probanden (p<0,05) und der unterdrückende Effekt von LgsBbBI auf die IL-5 Produtkion von SEA-stimulierten PBMC von gesunden Probanten war größer als bei allergischen Patienten (p<0,01, Figur 1).

### Genomische DNA von probiotischen Bakterien hemmen die Produktion von IL-4 und IL-5 durch SEA-stimulierte PBMC von gesunden Probanden in Abhängigkeit von der Zeit und der Dosierung.

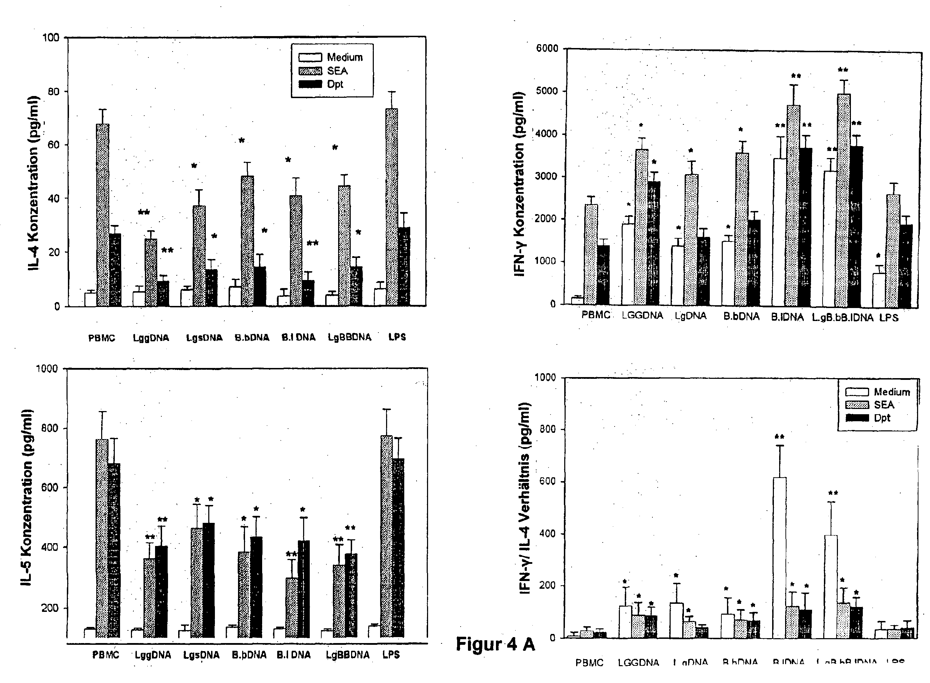

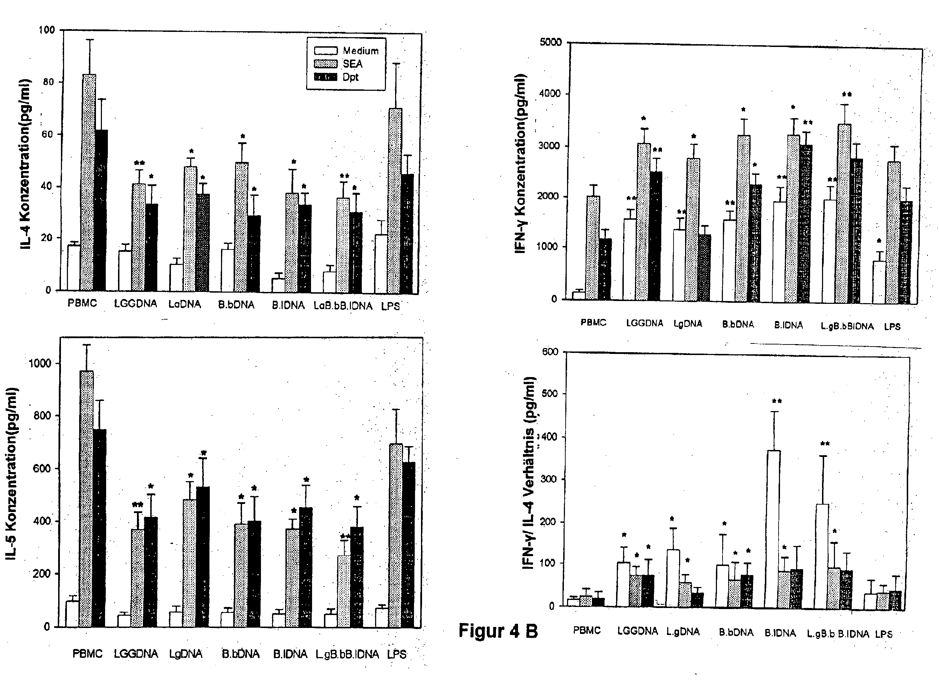

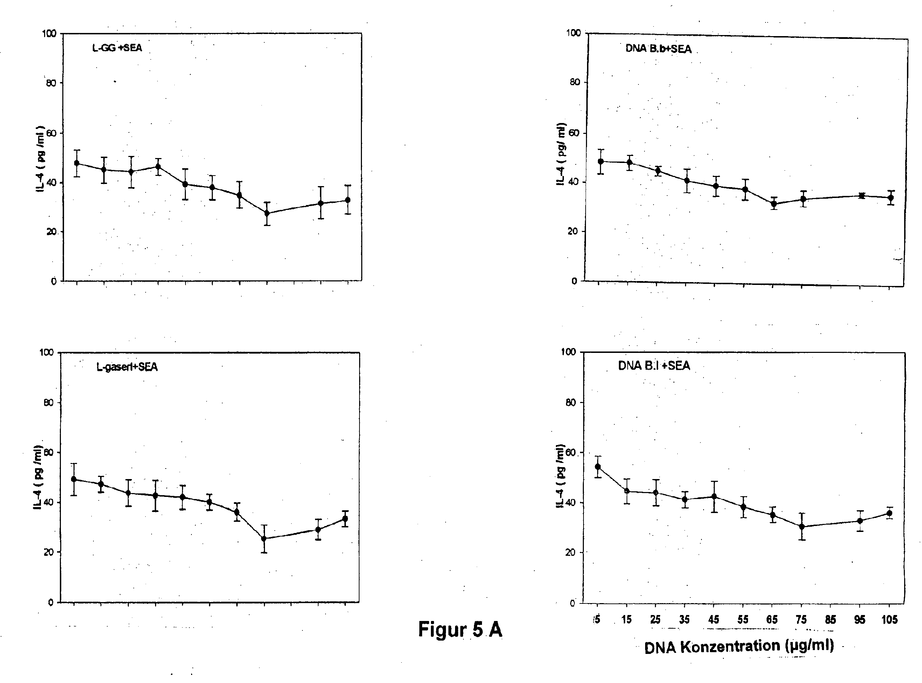

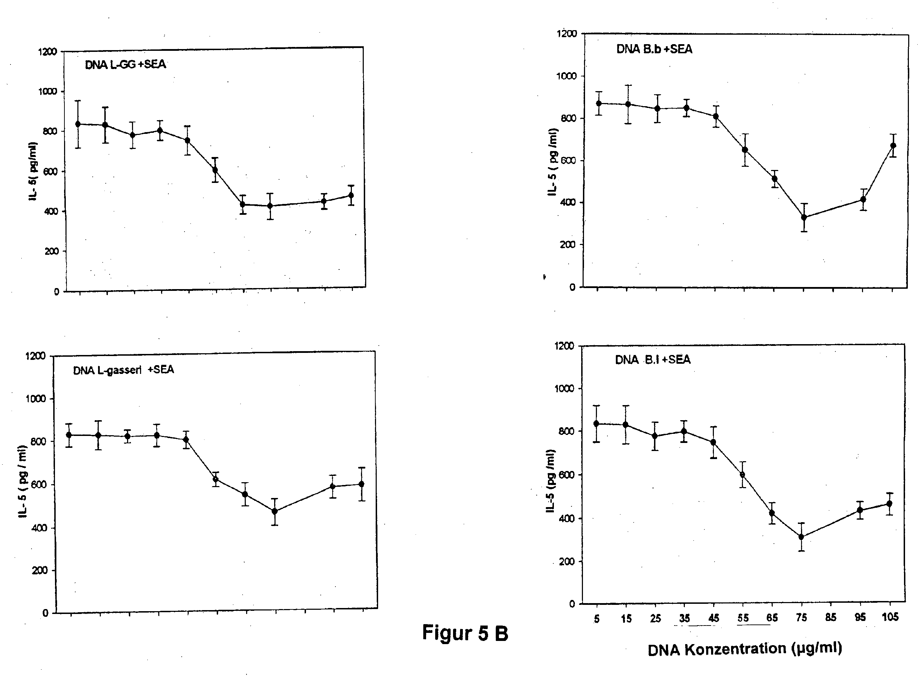

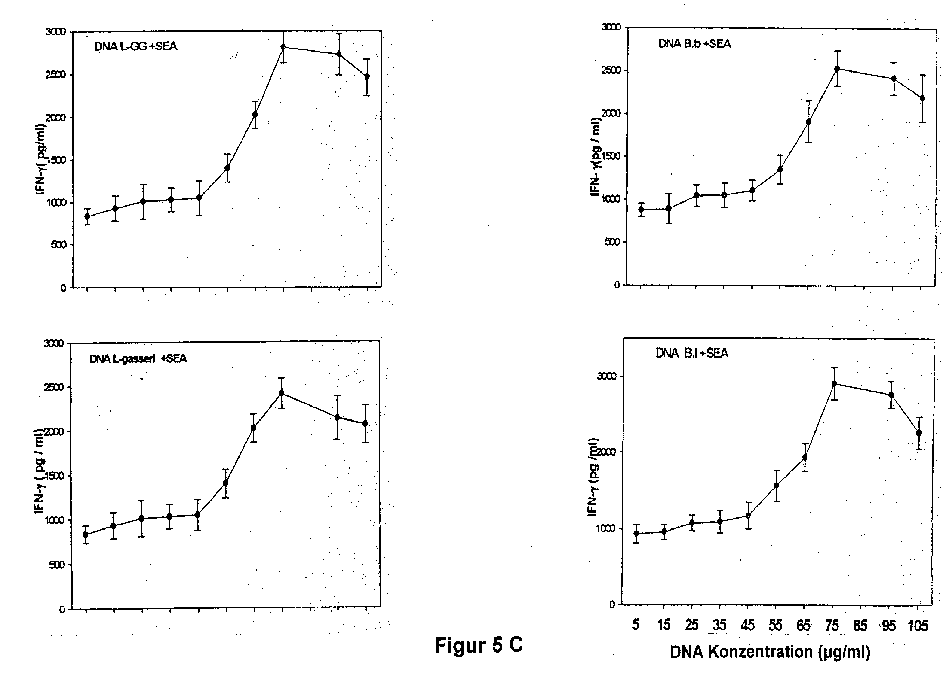

Um den wachstumshemmenden Effekt von bakterieller DNA in Vergleich zu tierischer DNA und LPS abzuschätzen wurden PBMCs von vier gesunden Probanden mit genomischer DNA von vier Stämmen probiotischer Bakterien, L-GG, L-gasseri, B.b, B.I and LgsBbBI (eine Mischung von L-gasseri, B.b and B.I), LPS und tierischer DNA (kalbs thymus DNA) inkubatiert. Die Unterdrückung von TH2 Cytokinen wurde überwacht durch die Anwendung von BDoptEIA Untersuchungssets um die Produktion von Cytokinen nachzuweisen und zu quantifizieren. Wie in Figur 4 dargestellt, hindert die DNA von probiotischen Bakterien normale PBMCs daran, IL-4 und IL-5 als Antwort auf die Stimulation durch SEA oder Dpt zu produzieren. Im Gegensatz dazu reduzierte LPS nicht die Produktion von IL-4 und IL-5 ebenso wie LPS-freie Kalbsthymus DNA die Produktion von IL-4 und IL-5 nicht vermindert (Daten nicht gezeigt).

### Zeitabhängige, wachstumshemmende Wirkung von bakterieller DNA auf die Produktion von IL-4 und IL-5 durch SEA-stimulierte PBMC von gesunden Probanten

Einige Experimente wurden durchgeführt um den zeitlichen Verlauf der Antwort auf genomische DNA zu bestimmen. PBMCs von gesunden Spendern (n = 4, bei 2x1 0⁶ml⁻¹) wurden mit SEA (2 µgml⁻¹) stimuliert und wurden entweder für eine, drei und sechs Stunden mit genomischer DNA von der L-GG oder B. b. (30 µgml⁻¹) vor dem SEA Stimulus vorinkubatiert oder zugleich mit genomischen DNAs stimuliert. Superantigene oder genomische DNAs wurden 1, 3 und 6 Stunden nach dem SEA Stimulus hinzugefügt und Zellkulturen wurden über eine Zeitdauer von 24, 48 und 72 Stunden inkubatiert. Die genomischen DNA von L-GG und B. b. wurde als repräsentativ für die beiden Lactobacillus- und Bifidobacteriumstämme ausgewählt. Zu den genannten Zeitpunkten wurden die zellfreien Überstände geerntet und nach Zentrifugierung und Sterilisation bei - 80°C gelagert. Die Konzentration von IL-4 und IL-5 wurde gemessen und es zeigte sich, dass die maximale Hemmung bei t₀ Stunden der Inkubation von PBMC mit genomischen DNAs und SEA (Daten nicht gezeigt) beobachtet wurde ebenso wie maximale Unterdrückung nach 24-stündiger Inkubation erreicht wurde. Diese Zeitdauer wurde bei weiteren Experimenten benutzt. Genomische DNA von B. b wurde als leistungsfähigerer Hemmer von IL-4 bestätigt als genomische DNA von L-GG. Im Gegensatz dazu wurde bestätigt, dass genomische DNA von L-GG ein leistungsfähigerer Hemmer von IL-5 ist als die genomische DNA von B. b.

### Dosierungsabhängiger, wachstumshemmender Effekt von bakterieller DNA auf die Produktion von IL-4 und IL-5 von SEA-stimulierten PBMCs von gesunden Probanten.

Um den wachstumshemmenden Effekt von bakterieller DNA auf die Hemmung von IL-4 und IL-5 durch SEA-stimulierte PBMC von gesunden Probanden zu untersuchen, wurde die genomische DNA von jedem Stamm in die PBMC-Kultur in verschiedenen Konzentrationen eingebracht, und zwar im Bereich von 5-105 µgml⁻¹, um zu bestimmen, welche Dosierung die größte hemmende Wirkung auf IL-4 und IL-5 Produktion hat (Figur 4). Dieser Dosierungsbereich wurde auf der Basis von zuvor beschriebenen Versuchsergebnissen gewählt, die zeigten, dass die Produktion von Cytokinen durch Immunzellen nach der Eingabe von wenigstens 3 µgml⁻¹ E-coli nachgewiesen werden konnte. DNA und die optimale Stimulation benötigten eine bakterielle DNA in einer Konzentration von >50 µgml⁻¹. Wie in Figur 4 gezeigt, wurde die maximale Hemmung von IL-4 und IL-5 dann beobachtet wenn genomische DNA in einer Konzentration von 75 µgml⁻¹ benutzt wurde. Drei verschiedene Muster von Cytokinantworten konnten klar unterschieden werden.

Genomische DNA: Alle Stämmen unterdrückten die Produktion von IL-4 und IL - 5 bei einer Konzentration von 75 µg ML⁻¹, mit Ausnahme von B. b-DNA und L-gg DANN, die die Produktion von EL -4 und EL-5 bei einer Konzentration von 65 µg ML⁻¹ unterdrücken.

Alle genomischen DNAs unterdrücken die Produktion IL - 4 und IL - 5 auf niedrigeren, stationären Pegeln, wenn sie in einem Konzentrationsbereich von 5 bis 45 µg ML⁻¹ zugegeben werden. Im Gegensatz dazu zeigten alle genomischen DNAs eine dosierungsabhängige Verstärkung der IL -4 und EL -5-Produktion, wenn sie in einem Konzentrationsbereich von 85 bis 105 µg ML⁻¹ angewendet wurden. Im Vergleich mit den anderen genomischen DNAs erscheinen B. b und L -gg DNAs leistungsfähigere Hemmer der IL -4 und IL -5-Produktion in Antwort auf eine SEA-Stimulation zu sein. Weiterhin zeigte die genomische DNA von B. b. den geringsten, hemmenden Einfluss auf IL-5-Produktion bei einer Konzentration von 105 µg ml⁻¹.

### Wachstumshemmende Wirkung von genomischer DNA von probiotischen Bakterien auf die Produktion von EL - 4 und EL - 5 durch SEA-stimulierte PBMC von allergischen Probanten.

Um die wachstumshemmende Wirkung von genomischer DNA von probiotischen Bakterien zu erforschen, wurde PBMC (2 x 10⁶ ml⁻¹) mit 75 µg ml⁻¹ von L -gg DNA, BI DNA und LgsBbBE DNA für 24 Stunden inkubatiert. Drei verschiedene Muster der Hemmung der IL -4-Produktion als Antwort auf die SEA-Stimulation konnten klar unterschieden werden: Genomische DNAs von L- G und LgsBBL zeigte den stärksten, hemmenden Effekt, nämlich 37,4 % +/- 4 % respektive 39,56 % +/- 5,1 %. L-Gasseri DNA zeigte den geringsten hemmenden Effekt (19,14 % +/- 2 %) und B.b.B.I. zeigten ein gleiches Muster (24,47 % +/- 3,28 %). Umgekehrt zeigte L-Gasseri DNA den höchsten, wachstumshemmenden Einfluss auf die IL-5-Produktion als Antwort auf SEA-Stimulation (61,41 % +/- 3,74 %), LgsBbBI DNA zeigte die geringste Unterdrückung (46,31 % +/- 4 %) und genomische DNA von L-GG, B.b. und B.i. zeigte ein gleiches Unterdrückungsmuster, (Daten nicht gezeigt). Weiterhin verglichen wir den hemmenden Effekt von genomischen DNAs mit lebenden Bakterien ebenso wie den unterdrückenden Effekt von genomischen DNA auf die IL- 4 und IL - 5-Produktion als Antwort auf die SEA-Stimulation zwischen gesunden und allergischen Probanden. Die wachstumshemmende Wirkung von lebenden Bakterien auf die IL-4-Produktion durch gesunde und allergische Probanden ist höher als der Einfluss ihrer genomischen DNA. Im Gegensatz dazu ist der wachstumshemmende Effekt von genomischer DNA in allergischen Probanden größer als in gesunden Probanten (mit Ausnahme von L-Gasseri). Folglich sind genomische DNAs leistungsfähiger bei der Reduzierung der IL -4-Produktion als Antwort auf die SEA-Stimulation bei allergischen Probanden.

Zusammenfassend gesagt war der wachstumshemmende Effekt von lebenden Bakterien auf die IL 5-Produktion als Antwort auf eine SEA-Stimulation bei PBMCs von gesunden und allergischen Patienten gleich wie der Effekt auf die IL -4-Produktion (z. B. war der hemmende Effekt von lebenden Bakterien höher als der ihrer genomischen DNA sowohl bei gesunden als auch allergischen Probanden). Aber die wachstumshemmende Wirkung von genomischen DNAs auf die IL-5-Produktion bei gesunden Spendern war größer als bei allergischen Probanden. Folglich sind genomische DNAs leistungsfähigere Hemmer für IL-5 in gesunden Spendern. In Bezug auf eine DPT-Stimulation war der hemmende Effekt von genomischer DNA von L-GG und LgsBbBI auf die IL-4-Produktion durch DPT stimulierte PBMC von allergischen Probanden höher als bei gesunden Probanden (p>0,05). Ein gleiches Muster wurde für genomische DNA von L-Gassery B. b und B. I. erhalten. Weiterhin war der hemmende Effekt von genomischer DNA von L-Gassery B.b B.I und LgsB.bB.I auf die IL 5-Produktion von DPT- stimulierter PBMC von gesunden Probanden höher als von allergischen Probanden. Aber der wachstumshemmende Einfluss von L-GG DNA auf die IL -5-Produktion bei gesunden Probanden war derselbe wie bei allergischen Probanden (Figur 3). Folglich kann die genomische DNA von L-GG die IL 5-Produktion von DPT-stimulierter PBMC von gesunden und allergischen Probanden in der gleichen Weise modulieren.

### Diskussion

Allergien sind hypersensitive Reaktionen des Immunsystems auf besondere Substanzen, die Allergene genannt werden (wie Pollen, Insekten, Gift, Arzneimittel oder Nahrungsmittel). Einige experimentelle Studien zeigen an, dass die allergischen Krankheiten in Verbindung gebracht werden könnten mit einer Störung der TH1/TH2 Cytokinebalance mit einer relativen Übergewichtung von TH2 Cytokinen und einem Fehlen von TH1 Cytokinen. In der Tat sind IL-4, IL-5, IL-3 und lL-9 an der Auslösung und der Aufrechterhaltung von allergischen Reaktionen beteiligt. Es wurde angenommen, dass TH2 Typ Cytokine bevorzugt in allergischen Krankheiten beteiligt sind, weil die "TH2 zu TH1 Immun-Antwort-Verschiebung" weithin sichtbar wird bei den meisten Typen von allergischen Erkrankungen. Die ermutigenden Ergebnisse bei der Behandlung von allergischen Patienten mit probiotischen Bakterien haben uns dazu veranlasst, die Wechselwirkung zwischen TH1/TH2 Cytokinen und Allergien zu erklären. Folglich ist eine Strategie bei der Therapie von Allergien die Veränderung der TH1/TH2 Balance durch die Eingabe von probiotischen Bakterien, um das TH1/TH2-Gleichgewicht wieder herzustellen. Von besonderem Interesse sind die Ergebnisse in Bezug auf die Fähigkeit von lebenden oder abgetöteten, probiotischen Bakterien, die Menge von IL-4 signifikant zu vermindern und die IFN-γ Cytokinmengen zu erhöhen. Weiterhin bewies eine aktuelle Studie, dass der schützende Effekt von probiotischen Bakterien durch die Freisetzung von löslichen Faktoren vermittelt werden könnte, die die Durchlässigkeit des Epithels ändern und gegen pathogene, bakterielle Invasionen schützen. Diese Daten erheben die Frage, ob dieser lösliche Faktor oder andere verschiedene Cytoplasmische Bakterienkomponenten, wie z. B. DNA, an der Auslösung und Modulation von Cytokinen beteiligt sein könnte. Aus diesem Blickwinkel erforschten wir die Auswirkung von lebenden Bakterien und reiner genomischer DNA von L-GG, L-gasseri, B. b, B.I. Stämmen und LgasBbBL (eine Mischung von L-gasserie B.b. und B.I.) auf die Freisetzung von den Cytokinen IL-4 und IL-5 unter Benutzung eines menschlichen Kulturmodelles. Die Ergebnisse zeigen, dass verschiedene Stämme von probiotischen Bakterien ebenso wie ihre genomischen DNA signifikante Wechsel im Profil der Cytokine auslösen, die in vitro von SEA oder Dptstimulierten PBMCs aktiv abgesondert werden. Weiterhin können sie die Th1/TH2 Balance modulieren indem sie die Produktion von TH2 Cytokinen vermindern und die von TH1 Cytokinen anwachsen lassen. Folglich können solche probiotischen Bakterien einen nützlichen Effekt bei allergischen Erkrankungen durch ihren hemmenden Effekt auf die Produktion von TH2 Cytokinen ausüben.

Um diesen Effekt zu untersuchen, haben wir zuerst PBMC von gesunden und allergischen Probanten mit SEA oder Dpt-stimuliert (als ein TH2-Cytokine-produzierendes Zellmodell). Zahlreiche Untersuchende haben berichtet, dass PBMCs TH2 Cytokine nach der Stimulation mit Superantigenen absondern. Wenn in dieser Studie SEA oder Dpt-stimulierte PBMCs mit lebenden, probiotischen Bakterien und ihren genomischen DNA vorinkubatiert wurden, wurde die Produktion von TH2 Cytokinen vermindert, aber nicht in der Gegenwart von E-coli. Zusätzlich war dieser hemmende Effekt abhängig von der Dosis, so dass in einer Konzentration von 2x10⁷ CFUml⁻¹ (entsprechend zu einem Verhältnis von 10:1 von Bakterien zu PBMC) der hemmende Effekt von lebenden Bakterien seinen Maximalwert in Bezug auf die IL-4 und IL-5 Produktion erreichte. Ein solcher dosierungsabhängiger Effekt wird in Vitro auch für die Produktion von IL-10 und IL-12 durch Monozyten von gesunden Probanden nach der Inkubation mit einigen Stämmen von Gram-positiven Bakterien berichtet. Diese Studie zeigte auch, dass nicht nur lebende probiotische Bakterien, sondern auch ihre genomische DNA die IL-4 und IL-5-Produktion durch SEA oder Dpt stimulierte PBMCs hemmen kann. Der wichtigste Punkt dieser Studie ist, dass erstmals eine vorteilhafte Wirkung von genomischer DNA oder probiotischen Bakterien auf die Zellen von allergischen Patienten vorgestellt wird, die auf Hausstaubmilben sensibilisiert sind; das sind mikroskopische Organismen, die in Wohnungen gefunden werden. Sie sind die vorrangige Ursache von Allergien in Bezug auf Staub.

Andere Berichte sind alle nur auf vorteilhafte Wirkungen von lebenden oder abgetöteten probiotischen Bakterien auf allergische Krankheiten bezogen, die von Nahrungsmittelallergien oder Aeroallergenen verursacht werden. Unsere Daten legen nahe, dass probiotische Bakterien und ihre genomische DNA durch direkte oder indirekte Regulation des Signalpfades wirken, der für die Unterdrückung von TH2 Cytokinen benötigt wird, weil der hemmende Effekt sogar dann beobachtet wurde, wenn die probiotischen Bakterien und ihre genomische DNA vor oder zugleich (für genomische DNA) mit der SEA oder Dpt Stimulation hinzugefügt wurden. Einige experimentelle Studien zeigen an, dass die Modulation der Produktion von IL-4 und IL-5 ein Multifaktor-Prozess ist und einige Cytokine wie z. B. IFN-γ als mögliche Regulatoren der IL-4 Produktion beschrieben werden. Weiterhin wird berichtet, dass IL-12 ein vorrangiges Cytokin bei der Auslösung der Produktion von IFN-γ durch menschliche PBMCs ist. In unserer Studie waren probiotische Bakterien und ihre genomische DNA in der Lage, die IL-12 Produktion durch PBMC zu steigern (Daten nicht gezeigt).

In dieser Beziehung wurde bakterielle DNA zu der SEA stimulierten PBMC-Kultur hinzugegeben und interessanter Weise zeigten diese Experimente eine manigfaltige IL-4 und IL-5 Cytokinproduktion nach der Stimulation mit bakterieller DNA und der SEA-Behandlung. Bakterielle DNA von probiotischen Bakterien reduzierte die IL-5-Absonderung stärker als die von IL-4. Unsere Daten legen nahe, dass genomische DNA von probiotischen Bakterien als Hemmer für die Produktion von IL-4 und IL-5 von SEA und Dpt stimulierten PBMC von gesunden und allergischen Probanden arbeiten können. Weiterhin berichtet eine aktuelle Studie, dass durch Hitze abgetötete, probiotische Bakterien die IL-4 und IL-5 Produktion durch SEA stimuliere PBMCs vermindern. Unter der Beachtung davon, dass der Herstellungsprozess, die Magen-Darm-Passage und hohe Temperaturen (70°C) die Lebensfähigkeit der Bakterien beeinflussen, zeigen die Ergebnisse dieser Studien einen möglichen hemmenden Effekt von genomischer DANN, die von durch Hitze getöteten Bakterien freigesetzt wird, welche zu PBMC hinzugegeben werden. Verschiedene Artikel beschreiben die Antwort von verschieden Cytokinen auf CPG-Leitelemente die in der bakteriellen DNA enthalten sind, wie z. B. genomische DNA von L-GG. Es wäre interessant zu wissen, ob die stammspezifische Freisetzung von Cytonkinen, die in dieser Studie demonstriert wurde, in Beziehung zu der genomischen Sequenz und den CPG-Leitelementen jedes Stammes steht.

Unserem Wissen nach ist dieser Bericht der erste, der immunomodulierende Wirkungen von genomischen DNAs von probiotischen Bakterien von SEA oder Dpt-stimulierten PBMCs von gesunden und allergischen Probanden zeigt. Die beobachteten Unterschiede bei Geschwindigkeit und Größe der Hemmung von IL-4 und IL-5 als Antwort auf bakterielle DNAs und die SEA oder Dpt-Stimulation sind interessante Informationen über den Einfluss von lebenden oder abgetöteten Probiotischen Bakterien und/oder bakteriellen Komponenten auf die Immunantwort. Das wachsende Wissen über Probiotik ist anregend, aber in naher Zukunft muss festgelegt werden, welche probiotische DNA (einzelne Stämme oder eine Kombination) die stärkste Wirkung bei besonderen Krankheiten hat. Gut organisierte, klinische Versuche nach dem Zufallsprinzip werden noch benötigt, um die Rolle von genomischen DNA von probiotischen Bakterien als Vorbeugungs- und Therapiemittel zukünftig zu definieren.

Im Folgenden sollen weitere Einzelheiten und Merkmale der Erfindungen anhand von Beispielen mit Ergebnissen der Versuchsreihen näher erläutert werden. Diese sollen die Erfindung jedoch nicht einschränken, sondern nur erläutern. Es zeigt in schematischer Darstellung:
**Figur 1**
   Zytokinmodulation von PBMC ohne (Medium) oder mit Stimulation von SEA und DPT.
**Figur 2**
   Dosierungsabhängiger, hemmender Effekt von lebenden probiotischen Bakterien auf die Produktion von IL - 4 (A), IL - 5 (B) und (IFN - γ) durch SEA-Stimulation der PBMC von gesunden Probanden.
**Figur 3**
   entfällt
**Figur 4**
   Wachstumshemmende Wirkung von genomischen DNA von probiotischen Bakterien auf die Produktion von IL - 4, IL -5 und IFN -γ durch SEA oder durch DPT stimulierte PBMC von gesunden (A, n=5) und allergischen (B, n=5) Probanden.
**Figur 5**
   Dosierungsabhängige, wachstumshemmende Wirkung von genomischer DNA auf die Produktion von IL-4 (A), IL-5 (B) und IFN-γ (C) durch PBMC von gesunden Probanden.

Die Figuren zeigen im Einzelnen:

### Figur 1

Zytokinmodulation von PBMC ohne (Medium) oder mit Stimulation von SEA und DPT.

PBMC's von gesunden (A, n=8) und allergischen (B, n=8) Probanden wurden vorinkubiert für 3 Stunden (bei 2 x 10⁶ Zellen/ml) mit Medium ohne (PBMC) oder mit vier Stämmen von probiotischen Bakterien, LgB.bB.L (eine Mischung von probiotischen Bakterien und einem Gram-negativem Kontrollbakterium (e-coli TG1) bei einem Bakterien zu Zellen Verhältnis von 10:1 vor entweder Stimulation mit dem SEA-Superantigen (2 µg/ML), DPT (2 µg/ML) oder nicht.

IL - 4, IL - 5 und IFN -γ wurde quantifiziert nach Stunden von Inkubation in Überständen durch eine spezifische ELISA-Untersuchung. Die Sterne zeigen eine besondere Unterdrückung oder Stimulation an (* p ≤ 0,05, **p ≤ 0,01) von TH₂ und TH₁ Zytokinproduktion im Vergleich mit der Kontrolle.

### Figur 2

Dosierungsabhängiger, hemmender Effekt von lebenden probiotischen Bakterien auf die Produktion von IL - 4 (A), IL - 5 (B) und (IFN - γ) durch SEA-Stimulation der PBMC von gesunden Probanden. PBMC von vier gesunden Spendern (2 x 10⁶ Zellen/ml⁻¹) wurde kultiviert mit vier Stämmen von probiotischen Bakterien, L-GG, L-Gassery, B. b. b. I. in Konzentrationen von 5 x 10⁴, 5 x 10⁵, 2 x 10⁶, 5 x 10⁶, 2 x 10⁷, 5 x 10⁷ entsprechend zu 0,025, 0,25, 1, 2,5, 5,10 und 25 Bakterien zu Zellenverhältnis für 3 Stunden vor der Stimulation mit 2 µg ml⁻¹ von SEA. Nach 48-stündiger Inkubation wurde IL -4 , IL -5 und IFN -γ mit spezifischer ELISA gemessen. Die Fehlerbalken stellen den Standardfehler des Mittelwertes dar.

### Figur 3

entfällt

### Figur 4

Wachstumshemmende Wirkung von genomischen DNA von probiotischen Bakterien auf die Produktion von IL - 4, IL -5 und IFN -γ durch SEA oder durch DPT stimulierte PBMC von gesunden (A, n=5) und allergischen (B, n=5) Probanden.

PBMC von fünf gesunden und fünf allergischen Probanden wurde für 48 Stunden (bei 2 x 10⁶ Zellen ml⁻¹) inkubatiert mit genomischer DNA von 4 Stämmen von probiotischen Bakterien, L-GG, L-Gasseri, B.b., B.I. und LGBBBL (eine Mischung von L-Gasseri, B.b und B. I.). Bei einer Konzentration von 75 µg ml⁻¹ vor der Stimulation mit SEA-(2 µg ML⁻¹) oder DPT (2000 SQ - EML ⁻¹ entsprechend einer 2 µg Gabe ml⁻¹).

Als Kontrolle wurden das Medium und LPS (100 ng ml⁻¹) benutzt. Die Produktion von IL-4, IL - 5 und IFN - γ wurde nach einer Inkubationszeit von 24 Stunden quantifiziert durch eine spezifische ELISA-Untersuchung. Die Sterne bezeichnen eine herausragende Hemmung (*p ≤ 0,05, **p ≤ 0,01) der Zytokinproduktion in Vergleich zu der Kontrolle (Medium). Die Daten sind ausgedrückt als der Mittelwert +/- SEM.

### Figur 5

Dosierungsabhängige, wachstumshemmende Wirkung von genomischer DNA auf die Produktion von IL-4 (A), IL-5 (B) und IFN-γ (C) durch PBMC von gesunden Probanden.

PBMC von 4 gesunden Spendern wurde kultiviert mit verschiedenen Konzentrationen (5 - 105 µg ml⁻¹) von bakterieller, genomischer DNA. Die Produktion von IL-4 (A) und IL-5 (B) wurde nach 24-stündiger Inkubation gemessen. Die Daten sind ausgedrückt als Mittelwert +/- SEM.

## Patentansprüche

1. Arzneimittel zur Anwendung zur Prophylaxe, Unterdrückung oder Eliminierung von allergischen Reaktionen bei Menschen, **dadurch**
**gekennzeichnet, dass** als wesentlicher Wirkbestandteil probiotische, Gram-positive Bakterien, nämlich die Bakterienstämme
- Lactobacillus gasseri PA 16/8 und/oder
- Bifidobacterium bifidum MG 20/5 und/oder
- Bifidobacterium longum SP 07/3
enthalten sind
und zwar als deren genomische DNA und/oder als lebensfähige und/oder inaktivierte Bakterien.

2. Arzneimittel zur Anwendung zur Verschiebung des TH1-TH-2-Verhältnisses im menschlichen Körper in Richtung eines Anstiegens von TH1 und/oder eines Abfalls von TH2
**dadurch gekennzeichnet, dass**
als wesentlicher Wirkbestandteil probiotischen, Gram-positiven Bakterien; nämlich die Bakterienstämme
- Lactobacillus gasseri PA 16/8 und/oder
- Bifidobacterium bifidum MG 20/5 und/oder
- Bifidobacterium longum SP 07/3
enthalten sind
und zwar als deren genomische DNA und/oder als lebensfähige und/oder inaktivierte Bakterien.

3. Verfahren zur Herstellung eines Arzneimittels zur Prophylaxe, Unterdrückung oder Eliminierung von allergischen Reaktionen bei Menschen
**gekennzeichnet durch** die Verwendung von probiotischen, Gram-positiven Bakterien, nämlich die Bakterienstämme
- Lactobacillus gasseri PA 16/8 und/oder
- Bifidobacterium bifidum MG 20/5 und/oder
- Bifidobacterium longum SP 07/3
und zwar als deren genomische DNA und/oder als lebensfähige und/oder inaktivierte Bakterien.

4. Verfahren zur Herstellung eines Arzneimittels zur Verschiebung des TH1-TH2-Verhältnisses im menschlichen Körper in Richtung eines Anstieges von TH1 und/oder eines Abfalls von TH2
**gekennzeichnet durch**
die Verwendung von probiotischen, Gram-positiven Bakterien, nämlich die Bakterienstämme
- Lactobacillus gasseri PA 16/8 und/oder
- Bifidobacterium bifidum MG 20/5 und/oder
- Bifidobacterium longum SP 07/3
und zwar als deren genomische DNA und/oder als lebensfähige und/oder inaktivierte Bakterien.

5. Erzeugnis bestehend aus probiotischen, Gram-positiven Bakterien, nämlich die Bakterienstämme
- Lactobacillus gasseri PA 16/8 und/oder
- Bifidobacterium bifidum MG 20/5 und/oder
- Bifidobacterium longum SP 07/3
und zwar als deren genomische DNA und/oder als lebensfähige und/oder inaktivierte Bakterien zur Anwendung zur Prophylaxe, Unterdrückung oder Eliminierung von allergischen menschlichen Reaktionen.

6. Erzeugnis bestehend aus probiotischen, Gram-positiven Bakterien, nämlich die Bakterienstämme
- Lactobacillus gasseri PA 16/8 und/oder
- Bifidobacterium bifidum MG 20/5 und/oder
- Bifidobacterium longum SP 07/3
und zwar als deren genomische DNA und/oder als lebensfähige und/oder inaktivierte Bakterien zur Anwendung zur Verschiebung des TH1-TH2-Verhältnisses im menschlichen Körper in Richtung eines Anstieges von TH1 und/oder eines Abfalls von TH2

7. Arzneimittel zur Anwendung gemäß einem der Ansprüche 1-2 oder 5-6, **dadurch gekennzeichnet, dass** es zur oralen Anwendung oder zur Verabreichung als Zäpfchen oder zur subcutanen Injektion oder zur intravenösen Injektion oder als Flüssigkeit zur Inhalation konfektioniert ist.

## Claims

1. Pharmaceutical for the prophylaxis, suppression, or elimination of allergic reactions in humans **characterised in that** probiotic, gram-positive bacteria are present as the substantial active ingredient, namely the bacterial strains
- Lactobacillus gasseri PA 16/8 and/or
- Bifidobacterium bifidum MG 20/5 and/or
- Bifidobacterium longum SP 07/3,
specifically as the genomic DNA thereof and/or as viable bacteria and/or as inactivated bacteria.

2. Pharmaceutical for shifting the Th1-Th2 balance in the human body towards an increase of Th1 and/or a decrease of Th2 **characterised in that** probiotic, gram-positive bacteria are present as the substantial active ingredient, namely the bacterial strains
- Lactobacillus gasseri PA 16/8 and/or
- Bifidobacterium bifidum MG 20/5 and/or
- Bifidobacterium longum SP 07/3
specifically as the genomic DNA thereof and/or as viable bacteria and/or as inactivated bacteria.

3. Method for preparing a pharmaceutical for the prophylaxis, suppression, or elimination of allergic reactions in humans, **characterised by**
the use of probiotic, gram-positive bacteria, namely the bacterial strains
- Lactobacillus gasseri PA 16/8 and/or
- Bifidobacterium bifidum MG 20/5 and/or
- Bifidobacterium longum SP 07/3
specifically as the genomic DNA thereof and/or as viable bacteria and/or as inactivated bacteria.

4. Method for producing a pharmaceutical for shifting the Th1-Th2 balance in the human body towards an increase of Th1 and/or a decrease of Th2, **characterised by**
the use of probiotic, gram-positive bacteria, namely the bacterial strains
- Lactobacillus gasseri PA 16/8 and/or
- Bifidobacterium bifidum MG 20/5 and/or
- Bifidobacterium longum SP 07/3
specifically as the genomic DNA thereof and/or as viable bacteria and/or as inactivated bacteria.

5. Product consisting of probiotic, gram-positive bacteria, namely the bacterial strains
- Lactobacillus gasseri PA 16/8 and/or
- Bifidobacterium bifidum MG 20/5 and/or
- Bifidobacterium longum SP 07/3
specifically as the genomic DNA thereof and/or as viable bacteria and/or as inactivated bacteria, for application for the prophylaxis, suppression or elimination of human allergic reactions.

6. Product consisting of probiotic, gram-positive bacteria, namely the bacterial strains
- Lactobacillus gasseri PA 16/8 and/or
- Bifidobacterium bifidum MG 20/5 and/or
- Bifidobacterium longum SP 07/3
specifically as the genomic DNA thereof and/or as viable bacteria and/or as inactivated bacteria, for use for shifting the Th1-Th2 balance in the human body towards an increase of Th1 and/or a decrease of Th2.

7. Pharmaceutical according to one of the previous claims, **characterised in that** it is prepared for oral use and/or for administration as suppositories or for subcutaneous injection or for intravenous injection or as a liquid for inhalation.

## Revendications

1. Médicament destiné à être utilisé en tant que prophylaxie, suppression ou élimination de réactions allergiques chez les personnes, **caractérisé par** le fait de contenir, en tant que composants actifs principaux, des bactéries probiotiques Gram-positives, à savoir les souches bactériennes
- lactobacillus gasseri PA 16/8 et/ou
- bifidobacterium bifidum MG 20/5 et/ou
- bifidobacterium longum SP 07/3,
à savoir en tant que leur ADN génomique et/ou en tant que bactéries viables et/ou inactivées.

2. Médicament destiné à être utilisé de façon à déplacer le rapport TH1-TH2 dans le corps humain en direction d'une augmentation du TH1 ou d'une baisse du TH2
**caractérisé par** le fait de contenir, en tant que composants actifs principaux, des bactéries probiotiques Gram-positives, à savoir les souches bactériennes
- lactobacillus gasseri PA 16/8 et/ou
- bifidobacterium bifidum MG 20/5 et/ou
- bifidobacterium longum SP 07/3,
à savoir en tant que leur ADN génomique et/ou en tant que bactéries viables et/ou inactivées.

3. Procédé destiné à fabriquer un médicament destiné à la prophylaxie, la suppression ou l'élimination de réactions allergiques chez les personnes, **caractérisé par** l'utilisation de bactéries probiotiques, Gram-positives, à savoir les souches bactériennes
- lactobacillus gasseri PA 16/8 et/ou
- bifidobacterium bifidum MG 20/5 et/ou
- bifidobacterium longum SP 07/3,
à savoir en tant que leur ADN génomique et/ou en tant que bactéries viables et/ou inactivées.

4. Procédé destiné à fabriquer un médicament destiné à déplacer le rapport TH1-TH2 dans le corps humain en direction d'une augmentation du TH1 ou d'une baisse du TH2 **caractérisé par le** fait de contenir, en tant que composants actifs principaux, des bactéries probiotiques Gram-positives, à savoir les souches bactériennes
- lactobacillus gasseri PA 16/8 et/ou
- bifidobacterium bifidum MG 20/5 et/ou
- bifidobacterium longum SP 07/3,
à savoir en tant que leur ADN génomique et/ou en tant que bactéries viables et/ou inactivées.

5. Produit consistant en bactéries probiotiques Gram-positives, à savoir les souches bactériennes
- lactobacillus gasseri PA 16/8 et/ou
- bifidobacterium bifidum MG 20/5 et/ou
- bifidobacterium longum SP 07/3,
à savoir en tant que leur ADN génomique et/ou en tant que bactéries viables et/ou inactivées destiné à être utilisé en tant que prophylaxie, suppression ou élimination de réactions allergiques chez les personnes.

6. Produit consistant en bactéries probiotiques Gram-positives, à savoir les souches bactériennes
- lactobacillus gasseri PA 16/8 et/ou
- bifidobacterium bifidum MG 20/5 et/ou
- bifidobacterium longum SP 07/3,
à savoir en tant que leur ADN génomique et/ou en tant que bactéries viables et/ou inactivées destiné à être utilisé de façon à déplacer le rapport TH1-TH2 dans le corps humain en direction d'une augmentation du TH1 ou d'une baisse du TH2.

7. Médicament destiné à être utilisé selon une des revendications 1-2 ou 5-6, **caractérisé par le fait qu'**il est confectionné pour une administration orale, ou une administration sous forme de suppositoire, ou pour injection sous-cutanée ou intraveineuse ou en tant que liquide pour inhalation.
